(19) Europäisches Patentamt
European Patent Office
Office européen des brevets

(11) Veröffentlichungsnummer: **0 137 395**
**B1**

(12) # EUROPÄISCHE PATENTSCHRIFT

(45) Veröffentlichungstag der Patentschrift: **04.11.87**

(51) Int. Cl.⁴: **C 07 C 149/36**, C 07 C 149/40, C 08 K 5/37

(21) Anmeldenummer: **84111363.2**

(22) Anmeldetag: **24.09.84**

(54) **Neue 4-(Hydroxyphenylthio)-benzoate.**

(30) Priorität: **26.09.83 US 536069**

(43) Veröffentlichungstag der Anmeldung: **17.04.85 Patentblatt 85/16**

(45) Bekanntmachung des Hinweises auf die Patenterteilung: **04.11.87 Patentblatt 87/45**

(84) Benannte Vertragsstaaten: **DE FR GB IT**

(56) Entgegenhaltungen:
**EP-A-0 079 855**
**US-A-3 457 286**
**US-A-4 120 846**
**US-A-4 394 476**

(73) Patentinhaber: **CIBA- GEIGY AG, Klybeckstrasse 141, CH-4002 Basel (CH)**

(72) Erfinder: **Spivack, John, Dr., 1 Blue Jay Street, Spring Valley New York 10977 (US)**
Erfinder: **Pastor, Stephen D., 112 Union Road, Spring Valley New York 10977 (US)**

(74) Vertreter: **Zumstein, Fritz, Dr., Bräuhausstrasse 4, D-8000 München 2 (DE)**

**EP 0 137 395 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue Merkaptophenolderivate, deren Verwendung als Stabilisatoren für organisches Material sowie die damit stabilisierten Zusammensetzungen.

Organische Polymere Verbindungen wie Kunststoffe oder Harze unterliegen thermischem, oxidativem oder lichtinduziertem Abbau. In der Technik ist eine grosse Anzahl von Stabilisatoren für eine Vielzahl von Substraten bekannt. Die Wirksamkeit eines Stabilisators hängt unter anderem von der Art des Substrats, in welchem er eingesetzt wird, und vom jeweiligen Abbaumechanismus ab. Daher ist es im allgemeinen schwierig, für eine konkrete Anwendung den wirksamsten und ökonomischsten Stabilisator anzugeben.

So kann beispielsweise ein Stabilisator, der die Flüchtigkeit einer Verbindung reduziert, dahingehend wirken, dass er einen Bindungsbruch der Substratmoleküle verhindert. Um eine geringe Versprödung oder die Erhaltung der Elastizität eines Polymeren oder eines Elastomeren zu gewährleisten, kann von einem Stabilisator verlangt werden, dass er übermässige Vernetzungsreaktionen und/oder Kettenbruch verhindert. Um die Vergilbung eines Substrats zu unterbinden, muss verhindert werden, dass Reaktionen des Substrats oder des Stabilisators ablaufen, die zu neuen Chromophoren führen. Weiterhin müssen Probleme der Verarbeitungsstabilität und der Substratverträglichkeit beachtet werden.

Überraschenderweise ist jetzt gefunden worden, dass die (Hydroxy-Phenylthio)-benzoate dieser Erfindung eine ungewöhnliche Kombination wünschenswerter Eigenschaften besitzen, wodurch diese Stoffe zu besonders effektiven Stabilisatoren werden. Die erfindungsgemässen Verbindungen erweisen sich als besonders nützliche Stabilisatoren für Polyolefine, schlagfestes Polystyrol, Elastomere wie Polybutadien oder Styrol-Butadien-Elastomere, bei denen der Erhalt der Elastizität und die Verhinderung von Vernetzungsreaktionen, von Versprödung, von Verfärbung, von Geruchsbildung und von Ausschwitzen des Stabilisators grundlegende Qualitätsanforderungen darstellen.

Eine Anzahl von Merkaptophenolderivaten ist bereits beschrieben worden. Viele dieser Verbindungen sind jedoch Ester von Hydroxyphenylthioalkancarbonsäuren.

Polyphenolische Ester von Hydroxyphenylthioalkancarbonsäuren sind in der US-PS-4 311 637 beschrieben.

Die Erfindung betrifft Verbindungen der Formel I

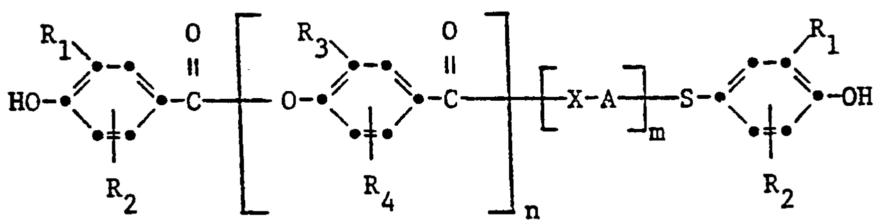 (I),

worin $R_1$, $R_2$, $R_3$ und $R_4$ unabhängig voneinander Wasserstoff, $C_1$-$C_{12}$ Alkyl, $C_5$-$C_6$ Cycloalkyl, Phenyl, durch $C_1$-$C_{12}$ Alkyl substituiertes Phenyl, $C_7$-$C_9$ Aralkyl oder durch $C_1$-$C_{12}$ Alkyl substituiertes $C_7$-$C_9$ Aralkyl bedeuten, worin

X -NH-, -O- oder -S- ist,

A $C_1$-$C_{10}$ Alkylen, Phenylen oder $C_5$-$C_6$ Cycloalkylen bedeutet, und worin

n und m unabhängig voneinander 0 oder 1 sind.

Bevorzugt werden Verbindungen der Formel I, bei denen die Reste $R_3$ und $R_4$ die gleiche Bedeutung besitzen wie die Reste $R_1$ und $R_2$ und bei denen sich die Reste $R_2$ und $R_4$ in o-Position zum Sauerstoffatom des Phenylrings befinden.

$R_1$, $R_2$, $R_3$ und $R_4$ sind als $C_1$-$C_{12}$ Alkyl beispielsweise Methyl, Ethyl, n-Propyl, Isopropyl, n-Butyl, sek.Butyl, tert.Butyl, tert.Amyl, n-Hexyl, 2-Ethylhexyl, n-Octyl, 1,1,3,3-Tetramethylbutyl, n-Decyl, n-Dodecyl und 1,1,3,3,5,5-Hexamethylhexyl. Bevorzugt werden $R_1$, $R_2$, $R_3$ und $R_4$ als $C_1$-$C_8$ Alkyl und besonders bevorzugt als tert.Butyl.

$R_1$, $R_2$, $R_3$ und $R_4$ bedeuten als $C_7$-$C_9$ Aralkyl beispielsweise Benzyl, α-Methylbenzyl oder α,α-Dimethylbenzyl. Als substituiertes Phenyl bedeuten diese Reste beispielsweise Tolyl, Mesityl oder Xylyl.

A kann als $C_1$-$C_{10}$ Alkylen geradkettig oder verzweigt sein. Bevorzugt wird $C_2$-$C_6$ Alkylen wie beispielsweise Ethylen, Trimethylen, Tetramethylen, 2,2-Dimethylpropan-1,3-diyl, Pentamethylen oder Hexamethylen. Bevorzugt wird Ethylen.

Als Cycloalkylen bedeutet A beispielsweise Cyclopentylen oder besonders bevorzugt Cyclohexylen.

X ist vorzugsweise Sauerstoff.

Bevorzugt werden Verbindungen der Formel I mit n = 1.

Zur Herstellung der Verbindungen der Formel I geht man von Zwischenprodukten der Formeln II, III, IV oder V aus. Hal bedeutet in diesen Formeln F, Cl, Br oder I. Die Zwischenprodukte werden mit Dialkyl-4-merkaptophenolen der Formel VI in an sich bekannter Weise umgesetzt (vgl. J.March, Advanced Organic Chemistry, McGraw-Hill, NY, 1977).

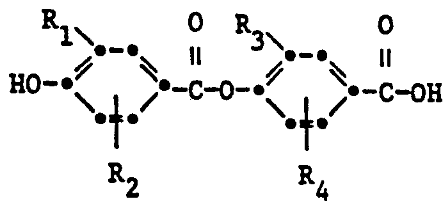

(II)

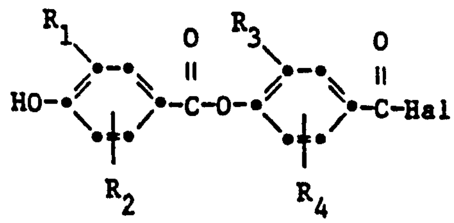

(III)

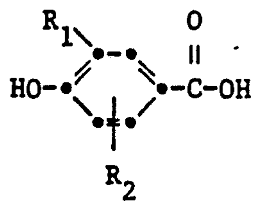

(IV)

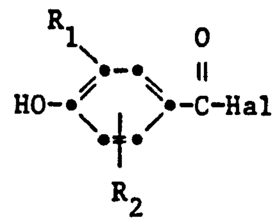

(V)

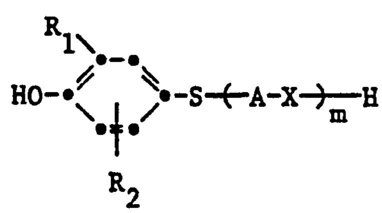

(VI)

Die Zwischenprodukte der Formel II oder der Formel III, bei denen sich die Reste $R_1$ und $R_2$ von den Resten $R_3$ und $R_4$ unterscheiden, können hergestellt werden, indem man das Chlorid einer Carbonsäure, die gegebenenfalls ein sterisch gehindertes Phenol enthält (beispielsweise 3,5-Di-tert.-butyl-4-hydroxybenzoylchlorid), mit einem geeignet alkylsubstituierten Hydroxybenzoat bei Temperaturen zwischen 50°C und 200°C umsetzt. Anschliessend hydrolysiert man den entstandenen Ester und überführt die entstandene Säure gegebenenfalls in an sich bekannter Weise in das Säurechlorid. Zwischenprodukte der Formel III, bei denen die Reste $R_1$ und $R_2$ die gleiche Bedeutung besitzen wie die Reste $R_3$ und $R_4$, können durch Reaktion von zwei Molen eines gegebenenfalls dialkylsubstituierten Hydroxybenzoylchlorids mit einem Mol einer Base erhalten werden.

Als Easen kann man Trialkylamine wie beispielsweise Triethylamin, Tri-n-propylamin, Tri-isopropylamin, Tri-n-butylamin, Tri-n-amylamin, Natrium- oder Kaliumhydroxyd oder vergleichbare Protonenakzeptoren verwenden.

Die Zwischenprodukte der Formeln II und III, bei denen die Reste $R_1$ und $R_2$ die gleiche Bedeutung wie die der Reste $R_3$ und $R_4$ besitzen können in der oben beschriebenen Weise hergestellt werden, indem man geeignet substituierte Ausgangsmaterialien einsetzt.

Zwischenprodukte der Formeln IV, V oder VI sind bekannte Verbindungen und als solche im Handel erhältlich oder nach an sich bekannten Verfahren herstellbar.

Die Synthese der Zwischenprodukte werden in inerten Lösungsmitteln durchgeführt. Beispiele dafür sind Kohlenwasserstoffe wie Hexan, Cyclohexan, Heptan oder chlorierte Kohlenwasserstoffe sowie Mineral-Öl und besonders bevorzugt aromatische Kohlenwasserstoffe wie beispielsweise Benzol oder Toluol.

Die Ausgangsmaterialien zur Herstellung der Zwischenprodukte II und III, bzw. der Verbindungen IV, V oder VI sind im Handel erhältlich oder können nach Standardmethoden erhalten werden.

Die erfindungsgemässen Verbindungen lassen sich als effektive Stabilisatoren für Kunststoffe, Polymere oder Harze einsetzen. Sie lassen sich auch als Stabilisatoren in Mineralölen oder synthetischen Ölen verwenden.

Die Erfindung betrifft daher auch eine Zusammensetzung enthaltend ein gegen oxidativen, thermischen oder strahlungsinduzierten Abbau empflindliches organisches Material und mindestens eine Verbindung der Formel I als Stabilisator, wobei als organisches Material vorzugsweise ein Polymer oder ein Schmierstoff auf Mineralölbasis oder ein synthetischer Schmierstoff zu verstehen ist, besonders bevorzugt aber ein polyolefinisches Homo- oder Copolymer, insbesondere aber auch ein Styrol Homopolymer, Copolymer oder Terpolymer.

Ein weiterer Gegenstand der Erfindung ist die Verwendung von Verbindungen der Formel I als Stabilisatoren für organisches Material gegen dessen Schädigung durch Einwirkung von Sauerstoff, Wärme, Luft oder energiereiche Strahlung.

Vorzugsweise verwendet man die Verbindungen der Formel I als Stabilisatoren für Polyolefine wie beispielsweise Polyethylen oder Polypropylen; Polystyrol, vorzugsweise schlagfestes Polystyrol; ABS-Harz; Elastomere wie beispielsweise Polybutadien, EPM, EPDM, SBR oder Nitril-Kautschuk; sowie Schmieröle, insbesondere solche auf Mineralölbasis. Schmiermittel, die mit den erfindungsgemässen Verbindungen stabilisiert werden können, sind dem Fachmann geläufig und beispielsweise im "Schmiermittel Taschenbuch" (Hüthig Verlag, Heidelberg, 1974) beschrieben.

Polymere, die mit den erfindungsgemässen Verbindungen stabilisiert werden können, sind beispielsweise:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z. B. von Cyclopenten oder Norbornen.

2. Mischungen der unter 1) genannten Polymeren, z. B. Mischungen von Polypropylen mit Polyisobutylen.

3. Copolymere von Mono- und Diolefinen untereinander oder mit anderen Vinylmonomeren, wie z. B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder x-Methylstyrol mit Dienen oder Acrylderivaten, wie z. B. Styrol-Butadien, Styrol-Acrylnitril, Styrol-Alkylmethacrylat, Styrol-Maleinanhydrid, Styrol-Acrylnitril-Methylacrylat; Mischungen von hoher Schlagzähigkeit aus Styrol-Copolymeren und einem anderen Polymer wie z. B. einem Polyacrylat, einem Dien-Polymeren oder einem Ethylen-Propylen-Dien-Terpolymeren; sowie Block-Copolymere des Styrols, wie z. B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z. B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z. B. als sogenannte ABS, MBS, ASA oder AES-Polymere bekannt sind.

7. Halogenhaltige Polymere, wie z. B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z. B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von $\alpha,\beta$-ungesättigen Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z. B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearet, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z. B. Ethylenoxid enthalten.

13. Polyphenyloxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylenisophthalamid, sowie deren Block-Copolymere mit Polyethern wie z. B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalet, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten.

18. Polycarbonate.
19. Polysulfone, Polyethersulfone und Polyetherketone.
20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.
21. Trocknende und nicht-trocknende Alkydharze.
22. Ungesättigte Polyesterherze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.
23. Vernetzbare Acrylharze, die sich von substituierten Acrylsäureestern ableiten wie z. B. von Epoxyacrylaten, Urethan-acrylaten oder Polyester-acrylaten.
24. Alkydharze, Polyesterharze und Acrylatharze, die mit Melaminharzen, Harnstoffharzen, Polyisocyanaten, oder Epoxidharzen vernetzt sind.
25. Vernetzte Epoxidharze, die sich von Polyepoxiden ableiten, z. B. von Bis-glycidylethern, von cycloaliphatischen Diepoxiden oder von aromatischen Diepoxiden.
26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.
27. Mischungen (Polyblends) der vorgenannten Polymeren wie z. B. PP/EPDM, Polyamid 6/EPDM oder ABS, PVC/EVA, PVC/ABS, PVC/MBS, PC/ABS, PBTP/ABS.
28. Natürliche und synthetische organische Stoffe, die reine monomere Verbindungen oder Mischungen von solchen darstellen, beispielsweise Mineralöle, tierische oder pflanzliche Fette, Öle und Wachse, oder Öle, Wachse und Fette auf Basis synthetischer Ester (z. B. Phthalate, Adipate, Phosphate oder Trimellithate), sowie Abmischungen synthetischer Ester mit Mineralölen in beliebigen Gewichtsverhältnissen, wie sie z. B. als Spinnpräparationen Anwendung finden, sowie deren wässrigen Emulsionen.
29. Wässrige Emulsionen natürlicher oder synthetischer Kautschuke, wie z. B. Naturkautschuk-Latex oder Latices von carboxylierten Styrol-Butadien-Copolymeren.

Die Erfindung betrifft weiterhin ein Verfahren zum Stabilisieren von organischem Material gegen oxidativen, thermischen oder strahlungsinduzierten Abbau, dadurch gekennzeichnet, dass man dem Material mindestens eine Verbindung der Formel I zusetzt.

Die erfindungsgemässen Stabilisatoren werden den Kunststoffen in Mengen von 0,01 bis 5 Gew.% bezogen auf das stabilisierte Material zugemischt. Je nach Verwendung und Substrat wird die Stabilisatormenge variiert. Bevorzugt verwendet man 0,05 bis 2 Gew.% des Stabilisators, besonders bevorzugt 0,1 bis 1 Gew.%.

Die Einarbeitung der Stabilisatorsubstanzen in die organischen Polymeren erfolgt nach bekannten Verfahren. Die Zudosierung kann während jeder Verarbeitungsstufe vor der Formgebung erfolgen. So kann der Stabilisator mit den pulverförmigen Polymeren gemischt werden, oder eine Emulsion bzw. eine Suspension des Stabilisators kann mit einer Lösung, Suspension oder Emulsion des Polymeren vermischt werden.

Die so stabilisierten Materialien können in verschiedenster Form angewendet werden, z. B. als Folien, Fasern, Bändchen, Formmassen, Profile oder als Bindemittel für Lacke, Klebstoffe oder Kitte.

In der Praxis können die Verbindungen der Formel I zusammen mit anderen Stabilisatoren eingesetzt werden.

Schmierstoff-Formulierungen können zusätzlich noch andere Additive enthalten, die zugegeben werden, um gewisse Gebrauchseigenschaften zu verbessern, wie z. B. weitere aminische Antioxidantien, Metallpassivatoren, Rostinhibitoren, Viskositäts-Index Verbesserer, Stockpunkterniedriger, Dispergiermittel/Tenside und Verschleisschutzadditive. Als weitere Additive, mit denen zusammen die erfindungsgemäss verwendeten Stabilisatoren eingesetzt werden können, sind beispielsweise zu nennen:

**1. Antioxidantien**

1.1. Alkylierte Monophenole
2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

1.2. Alkylierte Hydrochinone
2,6-Di-tert.butyl-4-methoxyphenol
2,5-Di-tert.butyl-hydrochinon

2,5-Di-tert.amyl-hydrochinon
2,6-Diphenyl-4-octadecyloxyphenol
2,5-Di-tert.butyl-hydrochinon
3,5-Di-tert.butyl-4-hydroxyanisol
Tris-(3,5-di-tert.butyl-4-hydroxyphenyl)-phosphit
3,5-Di-tert.butyl-4-hydroxyphenylstearat
Bis-(3,5-di-tert.butyl-4-hydroxyphenyl)-adipat

1.3. Hydroxylierte Thiodiphenylether
2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)
4,4'-Thio-bis-(3,6-di-sek.amylphenol)
4,4'-Bis-(2,6-dimethyl-4-hydroxyphenyl)-disulfid.

1.4. Alkyliden-Bisphenole
2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methlyen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercapto-butan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat
1,1-Bis-(3,5-dimethyl-2-hydroxyphenyl)-butan
2,2-Bis-(3,5-Di-tert.butyl-4-hydroxyphenyl)-propan
1,1,5,5-Tetra-(5-tert.butyl-4-hydroxy-2-methylphenyl)-pentan.

1.5. O-, N- und S-Benzyl Verbindungen
3,3,5,5-Tetra-tert.butyl-4,4-dihydroxydibenzylether
Octadecyl-4-hydroxy-3,5-dimethylbenzylmercaptoacetat
Tris-(3,5-di-tert.butyl-4-hydroxybenzyl)-amin
Bis-(4-tert.butyl-3-hydroxy-2,6-dimethylbenzyl)-dithioterephthalat.

1.6. Hydroxybenzylierte Malonate
Di-octadecyl-2,2-bis-(3,5-di-tert.butyl-2-hydroxybenzyl)-malonat
Di-octadecyl-2-(3-tert.butyl-4-hydroxy-5-methylbenzyl)-malonat
Di-dodecylmercaptoethyl-2,2-bis-(3,5-di-tert.butyl-4-hydroxybenzyl)-malonat
Di-[4-(1,1,3,3-tetramethylbutyl)-phenyl]-2,2-bis-(3,5-di-tert.butyl-4-hydroxybenzyl)-malonat.

1.7.Benzylverbindungen
1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz
1,4-Bis-(3,5-di-tert.butyl-4-hydroxybenzyl)-2,3,5,6-tetramethylbenzol
2,4,6-Tris-(3,5-di-tert.butyl-4-hydroxybenzyl)-phenol.

1.8. s-Triazinverbindungen

2,4-Bis-octylmercapto-6-(3,5-di-tert.butyl-4-hydroxyanilino)-s-triazin
2-Octylmercapto-4,6-bis-(3,5-di-tert.butyl-4-hydroxyanilino)-s-triazin
2-Octylmercapto-4,6-bis-(3,5-di-tert.butyl-4-hydroxyphenoxy)-s-triazin
2,4,6-Tris-(3.5-di-tert.butyl-4-hydroxyphenylethyl)-s-triazin
1,3,5-Tris-(3,5-di-butyl-4-hydroxyphenylethyl)-s-triazin
1,3,5-Tris-(3 5-di-tert.butyl-4-hydroxybenzyl)-isocyanurat.

1.9. Acylaminophenole
4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.10. Ester der β-(3,5-Di-tert.butyl-4-hydroxyphenyl)-propionsäure mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |
| Ethanol | 1,9-Nonadiol |
| Ethylenglykol | 1,2-Propandiol |
| 3-Thia-undecanol | 3-Thia-pentadecanol |
| Trimethylhexandiol | Trimethylolethan |
| Trimethylolpropan | |

4-Hydroxymethyl-1-phospha-2,6,7-trioxabicyclo-[2,2,2]-octan

1.11-Ester der β(5-tert.butyl-4-hydroxy-3-methylphenyl)-propion-säure mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |
| Ethanol | 1,9-Nonandiol |
| Ethylenglykol | 1,2-Propandiol |
| 3-Thia-undecanol | 3-Thia-pentadecanol |
| Trimethylhexandiol | Trimethylolethan |
| Trimethylolpropan | |

4-Hydroxymethyl-1-phospha-2,6,7-trioxabicyclo-(2,2,2)-octan

1.12. Amide der β-(3,5-Di-tert.butyl-4-hydroxyphenyl)-propionsäure, wie z. B.
N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin
N,N'-Di-(3,5-di-tert.butyl-4-hydroxyphenylpropionyl)-hexamethylendiamin
N,N-Bis-β-(3,5-di-tert.butyl-4-hydroxyphenylpropionyl)-isocyanurat
1.13. Ester der 3,5-Di-tert.butyl-4-hydroxyphenylessigsäure mit ein- oder mehrwertigen Alkoholen wie z. B.:

| | |
|---|---|
| Methanol | Ethanol |
| Octadecanol | 1,6-Hexandiol |
| 1,9-Nonandiol | Ethylenglycol |
| 1,2-Propandiol | Diethylenglycol |
| Thiodiglycol | Neopentylglycol |
| Pentaerithrit | 3-Thia-undecanol |
| 3-Thia-pentadecanol | Trimethylhexandiol |
| Trimethylolethan | Trimethylolpropan |
| Tris-hydroxyethyl-isocyanurat | |

4-Hydroxymethyl-1-phospha-2,6,7-trioxabicyclo-[2,2,2]-octan und besonders bevorzugt der Tetra-bis Ester mit Pentaerithrit.

1.14. Benzylphosphonate wie zum Beispiel:
Dimethyl-3,5-di-tert.butyl-4-hydroxybenzyl-phosphonat

Diethyl-3,5-di-tert.butyl-4-hydroxybenzyl-phosphonat
Dioctadecyl-3,5-di-tert.butyl-4-hydroxybenzyl-phosphonat
Dioctadecyl-5-tert.butyl-4-hydroxy-3-methylbenzyl-phosphonat.

Im folgenden sind Beispiele von weiteren Additiven aufgezählt, die zusammen mit den oben erwähnten Antioxidantien und den erfindungsgemässen Stabilisatoren eingesetzt werden können. Zu diesen Stoffen zählen beispielsweise:

**2. UV-Absorber und Lichtschutzmittel**

2.1. 2-(2'-Hydroxyphenyl)-benztriazole, wie z. B. das 5'-Methyl-, 3',5'-Di-tert.butyl-, 5'-Tert.butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-, 3'- -Methylbenzyl-5'-methyl-5-chlor-, 4'-Hydroxy-, 4'-Methoxy- 3'-Methyl-5'-carbomethoxyethyl-, 3',5'-Bis-( , -dimethylbenzyl)-5-chlor-, 3',5'-Di-tert.octyl-phenyl, 3',5'-Di-tert.-octylphenyl-5-chlor- oder 5-Chlor-3',5'-di-tert.amyl-Derivate.

2.2. 2,4-Bis-(2-hydroxyphenyl)-6-alkyl-s-triazine wie beispielsweise das
6-Ethyl-, 6-Heptadecyl- oder das 6-Undecyl-Derivat.

2.3. 2-Hydroxybenzophenone, wie z. B. das 4-Hydroxy-, 4-Methoxy-, 4-Octoxy- 4-Decyloxy-, 4-Dodecyloxy-, 4-Benzyloxy-, 4,2',4'-Tri-hydroxy- 2'-Hydroxy-4,4'-dimethoxy-Derivat.

2.4. 1,3-Bis-(2'-hydroxybenzoyl)-benzole wie beispielsweise:
1,3-Bis-(2'-hydroxy-4'-hexyloxybenzoyl)-benzol
1,3-Bis-(2'-hydroxy-4'-octyloxybenzoyl)-benzol
1,3-Bis-(2'-hydroxy-4'-dodecyloxybenzoyl)-benzol.

2.5. Ester von gegebenenfalls substituierten Benzoesäuren, wie z. B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester oder der n-Octadecylester oder der 2-Methyl-4,6-di-tert.butylester

2.6. Acrylate, wie z. B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäuremethylester, N-(β-Carbomethoxy-β-cyanovinyl)-2-methyl-indolin,

2.7. Nickelverbindungen, wie z. B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triethanolamin oder N-Cyclohexyl-diethanolanin, Nickeldibutyldithiocarbamat, Nickelsalze von 4-Hydroxy-3,5-di-tert.butylbenzylphosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.8. Stetisch gehinderte Amine, wis z. B. Bis-(2,2,6,6-tetramethylpiperidyl)-sebacat
Bis-(1,2,2,6,6-pentamethylpiperidyl)-sebacat
n-Butyl-3,5-di-tert.butyl-4-hydroxybenzyl-malonsäure-bis(1,2,2,6,6-pentamethylpiperidyl)-ester,
Kondensationsprodukt sus 1-Hydroxyethyl-2,2,6,6-Tetramethyl-4-hydroxypiperidin und Bernsteinsäure,
Kondensationsprodukt aus N,N'-(2,2,6,6-Tetramethylpiperidyl)-hexamethylendiamin und 4-tert.octylamino-2,6-dichlor-1,3,5-s-triazin,
Tris-(2,2,6,6-tetramethylpiperidyl)-nitrilotriacetat,
Tetrakis-(2,2,6,6-tetramethyl-4-piperidyl)-1,2,3,4-butantetracarbonsäure,
1,1'-(1,2-ethandiyl)-bis-(3,3,5,5-tetramethyl-piperazinon)
4-Benzoyl-2,2,6,6-tetramethylpiperidin, 4-Stearyl-oxy-2,2,6,6-tetramethylpiperidin, 3-n-Octyl-7-7-9-9-tetramethyl-1,3,5-triaza-spirs[4,5]-decan-2,4-dion.

2.9. Oxalsäurediamide, wie z. B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho- und para-Methoxy- sowie von o- und p-Ethoxy-di-substituierte Oxaniliden.

3. Metalldesaktivatoren, wie z. B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z. B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Isodecyloxy-2,4,8,10-tetraxya-3,9-diphospha-spiro-[5,5]-undecan und Tri-(4-hydroxy-3,5-di-tert.butylphenyl)-phosphit.

5. Peroxidzerstörende Verbindungen, wie z. B. Ester der β-Thio-dipropionsäure, beispielsweise der Lauryl-, Stearyl-, Myristyl- oder Tridecylester, Mercaptobenzimidazol, das Zinksalz des 2-Mercapto-benzimidazols, Zink-dibutyl-dithiocarbamat, Dioctadecyldisulfid, Pentaerythrit-tetrakis-(β-dodecylmercapto)-propionat.

6. Polyamidstabilisatoren, wie z. B. Kupfersalze in Kombination mit Jodien und/oder Phosphorverbindungen und Salze des zweiwertigen Mangans.

7. Basische Co-Stabilisatoren, wie z. B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z. B. 4-tert.Butylbenzoesäure, Adipinsäure, Diphenylessigsäure.

9. Füllstoffe und Verstärkungsmittel, wie z. B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z. B. Weichmacher, Gleitmittel, Emulgatoren, Pigmente, Optische Aufheller, Flammschutzmittel, Antistatika, Treibmittel.

11. Aminoaryl Derivate wie beispielsweise:
Phenyl-1-naphthylamin, Phenyl-2-naphthylamin, N,N'-Diphenyl-p-phenylendiamin, N,N'-Di-2-naphthyl-p-phenylendiamin, N,N'-Dinaphthyl-p-phenylendiamin, 6-Ethoxy-2,2,4-trimethyl-1,2-dihydrochinolin, 6-Dodecyl-2,2,4-trimethyl-1,2-dihydrochinolin, Mono- und Dooctyliminodibenzyl, polymerisiertes 2,2,4-Trimethyl-1,2-dihydrochinolin, octyliertes Diphenylamin, nonyliertes Diphenylamin, N-Phenyl-N'-cyclohexyl-p-phenylendiamin, N-Phenyl-N'-isopropyl-p-phenylendiamin, N,N'-Di-sek.octyl-p-phenylendiamin, N-Phenyl-N'-sek.octyl-p-phenylendiamin, N,N'-Di-(1,4-dimethylpentyl)-p-phenylendiamin, N,N'-Di-(1,4-dimethylpentyl)-p-phenylendiamin, N,N'-Dimethyl-N,N'-di-(sek.octyl)-p-phenylendiamin, 2,6-Dimethyl-4-methoxyanilin, 4-Ethoxy-N-sek.butylanilin, Kondensationsprodukt von Diphenylamin und Aceton, Aldol-1-naphthylamin und Phenothiazin.

12. Metallpassivatoren:
für Kupfer, z. B.:
Benztriazol, Tetrahydrobenztriazol, 2-Mercaptobenzthiazol, 2,5-Dimercaptothiadiazol, Salicylidenpropylendiamin, Salze von Salicylaminoguanidin.

13. Rost-Inhibitoren:
a) Organische Säuren, ihre Ester, Metallsalze und Anhydride, z. B.: N-Oleoyl-sarcosin, Sorbitan-mono-oleat, Blei-naphthenat, Dodecenylbernsteinsäure-anhydrid, Alkenylbernsteinsäure-Halbester, 4-Nonyl-phenoxyessigsäure.
b) Stickstoffhaltige Verbindungen, z. B.:
I. Primäre, sekundäre oder tertiäre aliphatische oder cycloaliphatische Amine und Amin-Salze von organischen und anorganischen Säuren. z. B. öllösliche Alkylammoniumcarboxylate.
II. Heterocyclische Verbindungen z. B.:
Substituierte Imidazoline und Oxazoline.
c) Phosphorhaltige Verbindungen, z. B.:
Aminsalze von Phosphorsäurepartialestern.
d) Schwefelhaltige Verbindungen, z. B.:
Barium-dinonylnaphthalin-sulfonate, Calciumpetroleum-sulfonate.

14. Viskositätsindex-Verbesserer:
Polymethacrylate, Vinylpyrrolidon/Methacrylat-Copolymere, Polybutene, Olefin-Copolymere, Styrol/Acrylat-Copolymere.

15. Stockpunktniedriger:
Polymethacrylat, alkylierte Naphthalinderivate.

16. Dispergiermittel/Tenside:

Polybutenylbernsteinsäure-imide, Polybutenylphosphonsäurederivate, basische Magnesium-, Calcium-, und Bariumsulfonate und -phenolate.

17. Verschleißschutz-Additive:
Schwefel und/oder Phosphor und/oder Halogen enthaltende Verbindungen, wie geschwefelte pflanzliche Öle, Zinkdialkyldithiophosphate, Tritolyl-phosphat, chlorierte Paraffine, Alkyl- und Aryldisulfide.

Die folgenden Beispiele erläutern die Erfindung. Alle Mengenangaben entsprechen Gewichtsteilen soweit nicht anderweitig angegeben.

**Beispiel 1**:

Herstellung von 2-(3,5-Di-tert.butyl-4-hydroxyphenylthio)-ethyl-[3,5-di-tert.butyl-4-(3,5-di-tert.butyl-4-hydroxybenzoyloxy)]-benzoat.

Zu einer Lösung aus 13,4 g 3,5-Di-tert.butyl-4-hydroxybenzoylchlorid und 100 ml trockenem Toluol werden bei 10 bis 15°C innerhalb von 10 Minuten 3,03 g Triethylamin in 20 ml Toluol getropft. Die Reaktionslösung wird anschliessend bei Raumtemperatur drei Stunden lang gerührt. Dann werden nochmals 3,03 g Triethylamin zugegeben und anschliessend innerhalb von 7 Minuten eine Lösung von 7,1 g 2-(3,5-Di-tert.butyl-4-hydroxyphenylthio)-ethanol in 30 ml Toluol zugefügt. Dabei wird die Temperatur bei 20 bis 25°C gehalten. Anschliessend erhitzt man die Reaktionslösung für 90 Minuten auf 60 bis 65°C und rührt weitere 15 Minuten bei Raumtemperatur aus. Das ausgefallene Triethylaminhydrochlorid wird abfiltriert und das Lösungsmittel anschliessend im Vakuum abgezogen. Der Rückstand wird aus Acetonitril umkristallisiert und man erhält 9,0 g eines weissen kristallinen Feststoffs entsprechend einer Ausbeute von 45 % d.Th. Die Kristalle schmelzen bei 168 bis 170°C.

| Analyse: | | C | H |
|---|---|---|---|
| | berechnet | 74,0 | 8,9 |
| | gefunden | 74,1 | 8,7 |

**Beispiel 2**:

Herstellung von S-(3,5-Di-tert.butyl-4-hydroxyphenyl)-3,5-di-tert.butyl-4-(3,5-di-tert.butyl-4-hydroxybenzoyloxy)-thiobanzoat.

Das Verfahren von Beispiel 1 wird wiederholt, indem man 16,1 g 3,5-Di-tert.butyl-4-hydroxybenzoylchlorid, 7,3 g 2,6-Di-tert.butyl-4-mercaptophenol und 7,1 g Triethylamin reagieren lässt. Der Rückstand wird aus einer 2-Propanol-Toluol-Mischung umkristallisiert und man erhält 15,6 g einer weissen Festsubstanz vom Schmelzpunkt 228 bis 230°C.

| Analyse: | | C | H |
|---|---|---|---|
| | berechnet | 75,2 | 8,9 |
| | gefunden | 75,1 | 8,9 |

**Beispiel 3**:

Herstellung von 2-(3,5-Di-tert.butyl-4-hydroxyphenylthio)-ethyl-3,5-di-tert.butyl-4-hydroxybenzoat.

Zu einer Lösung aus 9,8 g 2-(3,5-Di-tert.butyl-4-hydroxyphenylthio)-ethanol und aus 3,1 Pyridin in 40 ml Toluol wird bei 15 bis 20°C eine Lösung aus 9,4 g 3,5-Di-tert.butyl-4-hydroxybenzoylchlorid in 50 ml Toluol getropft. Die Zugabe dauert etwa 5 Minuten. Anschliessend wird 15 Stunden bei Raumtemperatur gerührt und dann das ausgefallene Triethylaminhydrochlorid abfiltriert. Das Lösungsmittel wird im Vakuum abgezogen und der Rückstand aus n-Heptan umkristallisiert. Man erhalt 15 g eines weissen Feststoffs vom F.P. 136 bis 139°C.

| Analyse: | | C | H |
|---|---|---|---|
| | berechnet | 72,3 | 8,9 |
| | gefunden | 72,2 | 8,7 |

**Beispiel 4**:

Herstellung von S-(3,5-bi-tert.butyl-4-hydroxyphenyl)-3 5-di-tert.butyl-4-hydroxythiobenzoat.

Das Verfahren von Beispiel 3 wird wiederholt, indem man 9,6 g 2,6-Di-tert.-butyl-4-merkaptophenol, 10,8 g 3,5-Di-tert.butyl-4-hydroxybenzoylchlorid und 3,6 g Pyridin miteinander umsetzt. Der Rückstand wird aus Acetonitril umkristallisiert und man erhält 8,8 g eines weissen Feststoffs entsprechend einer Ausbeute von 47 %. Das Produkt schmilzt bei 184 bis 186° C.

| Analyse: | | C | H |
|---|---|---|---|
| | berechnet | 74,0 | 9,0 |
| | gefunden | 73,9 | 8,7 |

**Beispiel 5**:

a) Herstellung von 1-(3,5-Di-tert.-butyl-4-hydroxyphenylthio)-1-hydroxymethan. Zu einer Lösung aus 28,6 g 2,6-Di-tert.-butyl-4-mercaptophenol und 9,74 g 37 %igem wässrigem Formaldehyd in 100 ml Methanol werden unter Rühren mehrere Tropfen einer 10 m Kalilauge zugefügt. Die Reaktionslösung wird 13 Stunden gerührt und die flüchtigen Bestandteile im Vakuum abgezogen. Der Rückstand wird aus Petrolether umkristallisiert und man erhält einen weissen Feststoff vom F.P. 79 bis 82° C.

| Analyse: | | C | H | S |
|---|---|---|---|---|
| | berechnet | 67,1 | 9,0 | 11,9 |
| | gefunden | 66,8 | 8,6 | 11,6 |

b) Herstellung von 3,5-Di-tert.-butyl-4-hydroxyphenylthiomethyl-(3,5-di-tert.-butyl-4-hydroxybenzoat).

Eine Lösung enthaltend 6,71 g des Reaktionsprodukts von Beispiel 5a, 6,26 g 2,6-Di-tert.-butyl-4-hydroxybenzoesäure, 6,56 g Triphenylphosphin und 4,36 g Diethylazodicarboxylat in 100 ml Tetrahydrofuran wird bei 40°C 24 Stunden lang gerührt. Das Lösungsmittel wird im Vakuum abgezogen und das Produkt chromatographisch gereinigt. Im IR-Spektrum ist eine Estercarbonylabsorptionsbande bei 1720 $cm^{-1}$ und eine phenolische Hydroxyabsorptionsbande bei 3610 $cm^{-1}$ zu erkennen.

**Beispiel 6**:

Stabilisierung von Polypropylen (Lichtstabilität)

Unstabilisiertes Polypropylenpulver (Hercules Profax ®6501) wird mit 0,2 Gew.% eines Additivs gemischt. Diese Mischung wird anschliessend bei 182°C 5 Minuten lang auf einem Mischwalzwerk plastifiziert. Anschliessend wird die stabilisierte Polypropylenfolie vom Walzwerk abgelöst und abkühlen gelassen. Die Folie wird in Stücke geschnitten und auf einer hydraulischen Presse bei 220° C und 12 bar zu einer 0,127 mm dicken Probe verformt. Diese Probe wird ultraviolettem Licht bis zur Zersetzung ausgesetzt. Als Zersetzungszeitpunkt wird diejenige Anzahl von Stunden gewertet, nach der die Probe die ersten Zerfallserscheinungen zeigt (braune Ecken, Risse). Die Messdaten sind in der folgenden Tabelle I dargestellt:

**Tabelle 1**:

| Additiv (Produkt von Beispiel) | Zahl der Stunden bis zur Zersetzung der bestrahlten Proben |
|---|---|
| 1 | 550 |
| 2 | 590 |
| 3 | 520 |
| 4 | 550 |
| - | 200-300 |

**Beispiel 7**:

Stabilisierung von Polypropylen (Oxidationsstabilität)

Um die Oxidationsstabilität von Polypropylen, das 0,2 Gew.% eines erfindungsgemässen Additivs oder einer

synergistisch wirkenden Formulierung bestehend aus 0,1 Gew.% eines Additivs und 0,3 Gew.% Distearyldithiodipropionat (DSTDP) enthält, zu untersuchen, werden Plättchen von 0,64 mm Dicke hergestellt und in einem Umluftofen bei 150°C gealtert. Als Zersetzungszeitpunkt wird diejenige Anzahl von Stunden gewertet, nach der die Probe die ersten Zerfallserscheinungen zeigt (braune Ecken, Risse).

Die Messdaten sind in Tabelle 2 dargestellt:

**Tabelle 2**:

| Additiv (Produkt von Beispiel) | Zahl der Stunden bis zur Zersetzung der Proben | |
|---|---|---|
| | Additiv | Additiv + DSTDP |
| 1 | 300 | 980 |
| 2 | 250 | 1200 |
| 3 | 70 | 110 |
| 4 | 60 | 90 |
| - | 20 | 20 |

**Beispiel 8**:

In diesem Beispiel wird die stabilisierende Wirkung der erfindungsgemässen Verbindungen in schlagfestem Polystyrol demonstriert.

a) Herstellung der Proben: Man stellt eine Lösung von 8 Gew.% Polybutadien-Kautschuk (Firestone DIENE 55) in monomerem Styrol her, indem man beide Komponenten in einer Quetschmühle mischt. Gleichzeitig wird in diesem Arbeitsgang der jeweils gewünschte Anteil des zu testenden Stabilisators zu der Lösung gegeben. Schliesslich fügt man noch 500 ppm Zn-Stearat hinzu, um später die polymerisierte Probe leicht aus dem Reaktionsgefäss ablösen zu können. Die Polymerisation wird in einem Gefäss durchgeführt, dass mit einem Wendelrührer ausgerüstet ist. Da die meisten IpS Blockpolymerisationen thermisch initiiert werden, wird in der vorliegenden Laboratoriumsversion kein Starter zugesetzt. Die Reaktion wird unter Stickstoff und unter Erwärmen durchgeführt. Der Reaktor wird innerhalb einer halben Stunde auf 121°C erwärmt, und diese Temperatur wird aufrecht erhalten, bis zwischen 30 und 35 % des Monomeren umgesetzt sind (Zeitdauer ca. 2,5 Stunden). Während der Polymerisation wird heftig gerührt, und die Rührgeschwindigkeit wird so eingestellt, dass Polymerpartikel von 2 bis 4 µm Grösse entstehen. Anschliessend werden die Reaktionsgefässe dem Reaktor entnommen, im Stickstoffstrom geöffnet und in ein Wirbelschichtsandbad gestellt, um die Polymerisation zu vervollständigen. Die Gefässe werden im Sandbad folgendermassen erhitzt: eine Stunde bei 100°C um die Anfangstemperatur vorzugeben, eine weitere Stunde, um 140°C zu erreichen und anschliessend weitere 8 Stunden, wobei die Temperatur jede Stunde um 10°C erhöht wird, bis schliesslich ein Maximum von 220°C erreicht ist. Nach dem Abkühlen werden die Polymerisationsgefässe zerbrochen und das Glas entfernt. Ein einziger Polymerblock wiegt im Mittel etwas über 600 g. Der Block wird in einem Vakuumofen bei 200°C für 45 Minuten bei einem Druck von 1,33 mbar belassen, um flüchtige Bestandteile zu entfernen. Nach dieser Behandlung wird der Block sofort in einer heizbaren hydraulischen Presse bei 205°C zwischen zwei Aluminiumfolien zu einem dicken Stab gepresst (drei Minuten Erhitzen, fünf Minuten ungeheizt).

Der Stab wird mittels einer Bandsäge zerkleinert und anschliessend granuliert. Alle Polymermischungen werden bei 205°C extrudiert und dann zerkleinert. Die Pellets werden unter Druck bei 205°C in dehnbare 3,175 mm dicke Streifen verformt. Diese Probestreifen werden dann bei 150°C in einem Umluftofen gealtert, wobei sie auf Glasplatten, die sich wiederum auf rotierenden Trägern befinden, aufgebracht sind. Eine weitere Charge von Probestreifen wird auf dieselbe Weise bei 80°C im Ofen gealtert. Nach bestimmten Zeitintervallen wird der Yellowness Index der Proben gemäss ASTM D-1925-63T bestimmt, und es wird die Dehnbarkeit bestimmt (Instron Tensile Testing Apparatus, Instron Engineering Corporation, Massachusetts; Ziehgeschwindigkeit: 5 mm/Minute).

In der folgenden Tabelle 3a sind die Ergebnisse der Dehnbarkeitsmessungen und der Messungen des Yellowness-Index von bei 80°C ofengealterten Proben angegeben, während Tabelle 3b Messwerte von bei 150°C ofengealterten Proben enthält.

**Tabelle 3a**:

Dehnbarkeitsmessungen und Yellowness Index von bei 80°C ofengealterten Proben mit Stabilisator

| Additiv (Produkt von Beispiel) | Stabilisator-menge (Gew.-%) | Dehnbarkeit der Proben (%) und Dauer der Hitzeeinwirkung (h) | | | |
|---|---|---|---|---|---|
| | | 0 h | 300 h | 600 h | 900 h |
| 2 | 0,1 | 23 | 19 | - | - |
| 4 | 0,1 | 52 | 28 | 11 | 7 |
| - | - | 33 | 9 | 3 | 3 |

| Additiv (Produkt von Beispiel) | Stabilisator-menge (Gew.-%) | Yellowness Index und Dauer der Hitzebelastung (h) | | | |
|---|---|---|---|---|---|
| | | 0 h | 300 h | 600 h | 900 h |
| 2 | 0,1 | 0 | 20 | 29 | 52 |
| 4 | 0,1 | 1 | 12 | 15 | 32 |
| - | - | 7 | 14 | 45 | 59 |

**Tabelle 3b**:

Dehnbarkeitsmessungen und Yellowness Index von bei 150° C ofengealterten Proben mit Stabilisator

| Additiv (Produkt von Beispiel | Stabilisator-menge (Gew.-%) | Dehnbarkeit der Probe (%) und Dauer der Hitzebelastung (h) | | | | |
|---|---|---|---|---|---|---|
| | | 0 h | 0,5 h | 1 h | 1,5 h | 2 h |
| 2 | 0,1 | 23 | 16 | 13 | 7 | 7 |
| 4 | 0,1 | 52 | 32 | 8 | 7 | 7 |
| - | - | 33 | 7 | 7 | 3 | 3 |

| Additiv (Produkt von Beispiel | Stabilisator-menge (Gew.-%) | Yellowness Index und Dauer der Hitzebelastung (h) | | | | |
|---|---|---|---|---|---|---|
| | | 0 h | 0,5 h | 1 h | 1,5 h | 2 h |
| 2 | 0,1 | 0 | 2 | 5 | 8 | 14 |
| 4 | 0,1 | 1 | 4 | 5 | 8 | 14 |
| - | - | 7 | 18 | 30 | 38 | 43 |

**Patentansprüche**

1. Verbindungen der Formel I

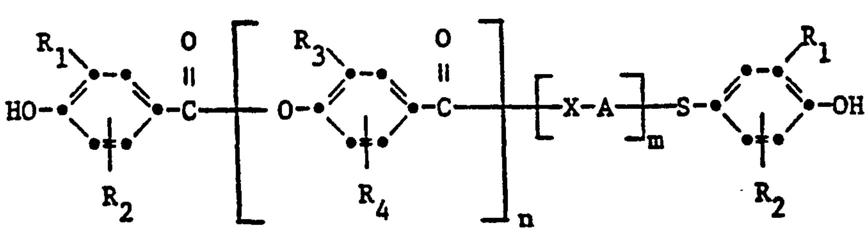 (I),

worin $R_1$ $R_2$, $R_3$ und $R_4$ unabhängig voneinander Wasserstoff, $C_1$-$C_{12}$-Alkyl, $C_5$-$C_6$ Cycloalkyl, Phenyl, durch $C_1$-$C_{12}$ Alkyl substituiertes Phenyl, $C_7$-$C_9$ Aralkyl oder durch $C_1$-$C_{12}$ Alkyl substituiertes $C_7$-$C_9$-Aralkyl bedeuten, worin
X -NH-, -O- oder -S- ist,
A $C_1$-$C_{10}$ Alkylen, Phenylen oder $C_5$-$C_6$ Cycloalkylen bedeutet, und
worin
n und m unabhängig voneinander 0 oder 1 sind.

2. Verbindungen der Formel I gemäss Anspruch 1, bei denen die Reste $R_3$ und $R_4$ die gleiche Bedeutung besitzen wie die Reste $R_1$ und $R_2$ und bei denen sich die Reste $R_2$ und $R_4$ in o-Position zum Sauerstoffatom des

Phenylrings befinden.

3. Verbindungen der Formel I gemäss Anspruch 2, bei denen die Reste $R_1$ und $R_2$ $C_1$-$C_8$ Alkyl bedeuten.

4. Verbindungen der Formel I gemäss Anspruch 2, bei denen die Reste $R_1$ und $R_2$ tert.Butyl bedeuten.

5. Verbindungen der Formel I gemäss Anspruch 1, bei denen A $C_2$-$C_6$ Alkylen bedeutet.

6. Verbindungen der Formel I gemäss Anspruch 1, bei denen A Ethylen bedeutet.

7. Verbindungen der Formel I gemäss Anspruch 1, bei denen X Sauerstoff bedeutet.

8. Verbindungen der Formel I gemäss Anspruch 1, bei denen n 1 bedeutet.

9. Die Verbindungen 2-(3,5-Di-tert.butyl-4-hydroxyphenylthio)-ethyl-[3,5-di-tert.butyl-4-(3,5-di-tert.butyl-4-hydroxybenzoyloxy)-benzoat], S-(3,5-Di-tert.butyl-4-hydroxyphenyl)-(3,5-di-tert.butyl-4-(3,5-di-tert.butyl-4-hydroxybenzoyloxy)-thiobenzoat), 2-(3,5-Di-tert.butyl-4-hydroxyphenylthio)-ethyl-(3,5-di-tert.butyl-4-hydroxybenzoat) oder S-(3,5-Di-tert.butyl-4-hydroxyphenyl)-(3,5-di-tert.butyl-4-hydroxythio-benzoat) gemäss Anspruch 1.

10. Eine Zusammensetzung enthaltend ein gegen oxidativen thermischen oder strahlungsinduzierten Abbau empfindliches organisches Material und mindestens eine Verbindung der Formel I gemäss Anspruch 1 als Stabilisator.

11. Eine Zusammensetzung gemäss Anspruch 10, worin unter dem organischen Material ein synthetisches Polymer zu verstehen ist.

12. Eine Zusammensetzung gemäss Anspruch 11, worin unter dem synthetischen Polymer ein Polyolefin, schlagfestes Polystyrol, ABS-Harz, Butadienkautschuk, EPM, EPDM, SBR oder Nitrilkautschuk zu verstehen ist.

13. Ein Verfahren zum Stabilisieren von organischem Material gegen oxidativen, thermischen oder strahlungsinduzierten Abbau, dadurch gekennzeichnet, dass man dem Material mindestens eine Verbindung der Formel I gemäss Anspruch 1 zusetzt.

14. Verwendung von Verbindungen der Formel I gemäss Anspruch 1 als Stabilisatoren für organisches Material gegen dessen Schädigung durch Einwirkung von Sauerstoff, Wärme oder energiereiche Strahlung.

## Claims

1. Compunds of the formula I

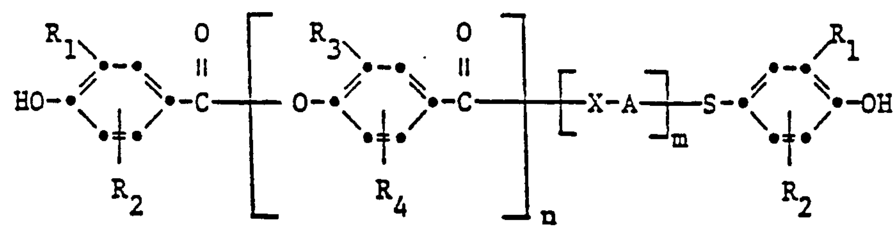

(I)

wherein

$R_1$, $R_2$, $R_3$ and $R_4$ are independently of each other hydrogen, $C_1$-$C_{12}$-alkyl, $C_5$-$C_6$-cycloalkyl, phenyl, phenyl substituted by $C_1$-$C_{12}$-alkyl, $C_7$-$C_9$-aralkyl or aralkyl substituted by $C_1$-$C_{12}$-alkyl;

X is -NH-, -O- or -S-;

A is $C_1$-$C_{10}$-alkylene, phenylene or $C_5$-$C_6$-cycloalkylene and

n and m independently of each other are 0 or 1.

2. Compounds of the formula I according to claim 1, wherein the radicals $R_3$ and $R_4$ have the same meanings as the radicals $R_1$ and $R_2$ and the radicals $R_2$ and $R_4$ are in the ortho position relative to the oxygen atom of the phenyl ring.

3. Compounds of the formula I according to claim 2, wherein the radicals $R_1$ and $R_2$ are $C_1$-$C_8$-alkyl.

4. Compounds of the formula I according to claim 2, wherein the radicals $R_1$ and $R_2$ are tert-butyl.

5. Compounds of the formula I according to claim 1, wherein A is $C_2$-$C_6$-alkylene.

6. Compounds of the formula I according to claim 1, wherein A is ethylene.

7. Compounds of the formula I according to claim 1, wherein X is oxygen.

8. Compounds of the formula I according to claim 1, wherein n is 1.

9. The compounds 2-(3,5-di-tert-butyl-4-hydroxyphenyl-thio)ethyl 3,5-di-tert-butyl-4-(3,5-di-tert-butyl-4-hydroxybenzoyloxy)benzoate, S-(3,5-di-tert-butyl-4-hydroxyphenyl) 3,5-di-tert-butyl-4-(3,5-di-tert-butyl-4-hydroxybenzoyloxy)thiobenzoate, 2-(3,5-di-tert-butyl-4-hydroxyphenylthio)ethyl 3,5-di-tert-butyl-4-hydroxybenzoate or S-(3,5-di-tert-butyl-4-hydroxyphenyl) 3,5-di-tert-butyl-4-hydroxythiobenzoate according to claim 1.

10. A composition containing an organic material sensitive to oxidative, thermal or radiation-induced degradation and at least one compound of the formula I according to claim 1 as stabilizer.

11. A composition according to claim 10, wherein the organic material is a synthetic polymer.

12. A composition according to claim 11, wherein the synthetic polymer is a polyolefin, impact polystyrene, ABS resin, butadiene rubber, EPM, EPDM, SBR or nitrile rubber.

13. A method for stabilizing organic material against oxidative, thermal or radiation-induced degradation which comprises adding at least one compound of the formula I according to claim 1 to the material.

14. Use of compounds of the formula I according to claim 1 as stabilizers for organic material against damage by action of oxygen, heat or high-energy radiation.

**Revendications**

1. Composés répondant à la formule I:

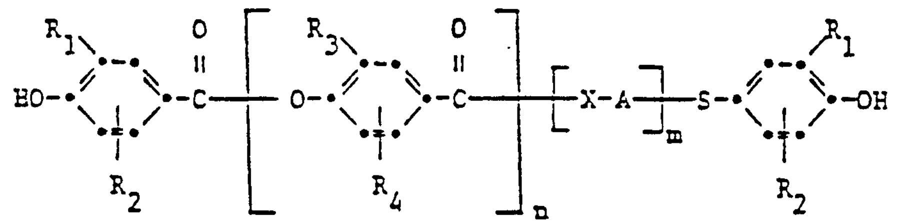

(I)

dans laquelle

$R_1$, $R_2$, $R_3$ et $R_4$ représentent chacun, indépendamment les uns des autres, l'hydrogène, un alkyle en $C_1$-$C_{12}$, un cycloalkyle en $C_5$ ou $C_6$, un phényle, un phényle porteur d'un alkyle en $C_1$-$C_{12}$, un aralkyle en $C_7$-$C_9$ ou un aralkyle en $C_7$-$C_9$ porteur d'un alkyle en $C_1$-$C_{12}$,

X représente -NH-, -O- ou -S-,

A représente un alkylène en $C_1$-$C_{10}$, un phénylène ou un cycloalkylène en $C_5$ ou $C_6$ et

n et m représentent chacun, indépendamment l'un de l'autre, le nombre 0 ou le nombre 1.

2. Composés de formule I selon la revendication 1 dans lesquels les symboles $R_3$ et $R_4$ ont les même significations que les symboles $R_1$ et $R_2$ et dans lesquels les radicaux $R_2$ et $R_4$ occupent, chacun sur son noyau phényle, une position ortho par rapport à l'atome d'oxygène.

3. Composés de formule I selon la revendication 2 dans lesquels $R_1$ et $R_2$ représentent chacun un radical alkyle contenant de 1 à 6 atomes de carbone.

4. Composés de formule I selon la revendication 2 dans lesquels $R_1$ et $R_2$ représentent chacun un radical tertbutyle.

5. Composés de formule I selon la revendication 1 dans lesquels A représente un radical alkylène contenant de 2 à 6 atomes de carbone.

6. Composés de formule I selon la revendication 1 dans lesquels A représente un radical éthylène.

7. Composés de formule I selon la revendication 1 dans lesquels X représente l'oxygène.

8. Composés de formule I selon la revendication 1 dans lesquels n est égal à 1.

9. Composés selon la revendication 1, en l'espèce:

- le di-tert-butyl-3,5 (di-tert-butyl-3,5 hydroxy-4 benzoyloxy)-4 benzoate de (di-tert-butyl-3,5 hydroxy-4 phénylthio)-2 éthyle,
- le di-tert-butyl-3,5 (di-tert-butyl-3,5 hydroxy-4 benzoyloxy)-4 thiobenzoate de S-(di-tert-butyl-3,5 hydroxy-4 phényle,
- le di-tert-butyl-3,5 hydroxy-4 benzoate de (di-tert-butyl-3,5 hydroxy-4 phénylthio)-2 éthyle et
- le di-tert-butyl-3,5 hydroxy-4 thiobenzoate de S-(di-tert-butyl-3,5 hydroxy-4 phényle).

10. Composition contenant une matière organique risquant de subir une dégradation sous l'effet de l'oxydation, de la chaleur ou d'un rayonnement et au moins un composé de formule I selon la revendication 1 comme stabilisant.

11. Composition selon la revendication 10 dans laquelle la matière organique est un polymère synthétique.

12. Composition selon la revendication 11 dans laquelle le polymère synthétique est une polyoléfine, le polystyrène choc, la résine ABS, le caoutchouc de butadiène, l'EPM, l'EPDM, le SBR ou le caoutchouc nitrile.

13. Procédé pour stabiliser des matières organiques contre la dégradation que pourraient leur occasionner l'oxydation, la chaleur ou des rayonnemente, procédé caractérisé en ce qu'on ajoute à la matière au moins un composé de formule I selon la revendication 1.

14. Application de composés de formule I selon la revendication 1 comme stabilisants pour des matières organiques à protéger contre les dommages que risque de leur occasionner l'action de l'oxygène, de la chaleur ou d'un rayonnement de haute énergie.

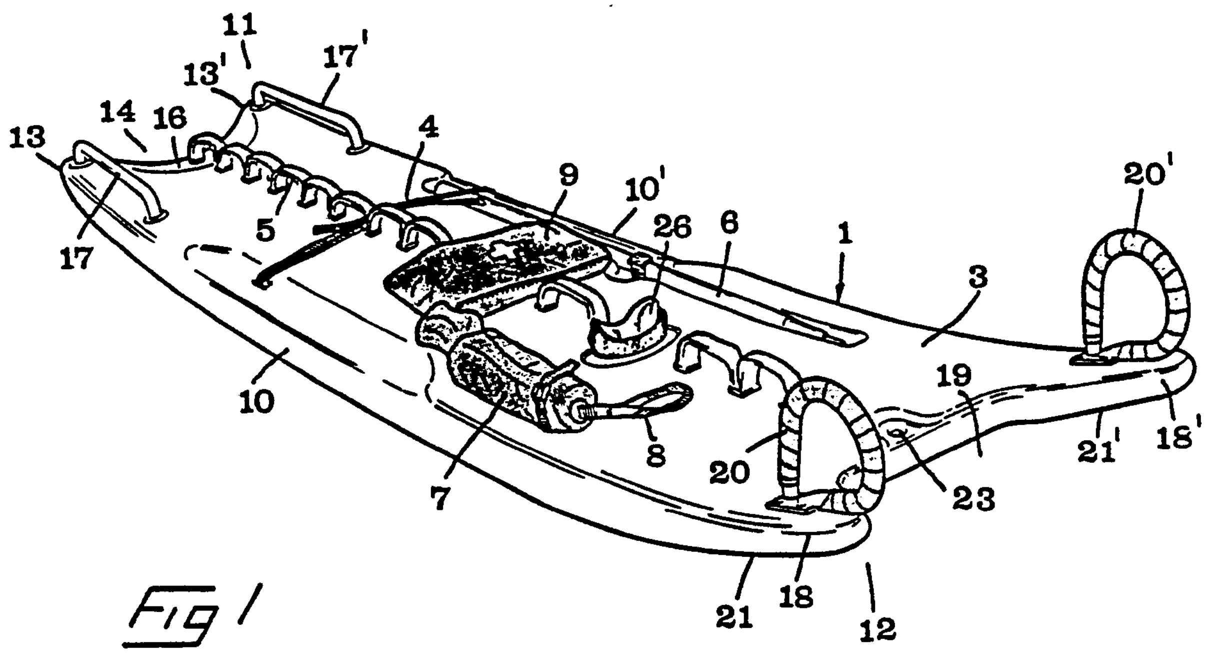
11
13'
17'
14
16
13
4
9
10'
20'
26
6
1
3
5
17
19
10
8
20
18'
21'
23
7
21
18
12

Fig 1

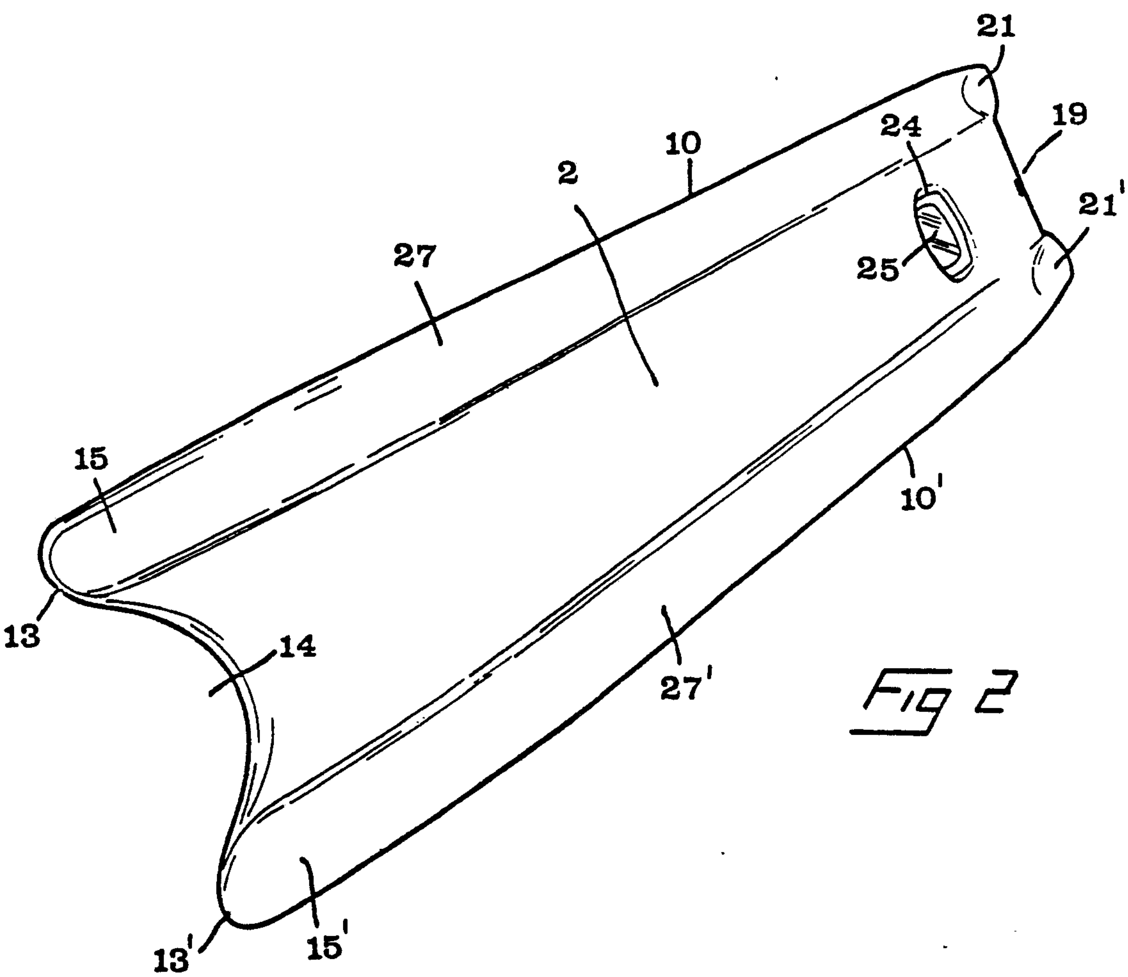
21
19
24
10
2
21'
25
27
15
10'
13
14
27'
13'
15'

Fig 2

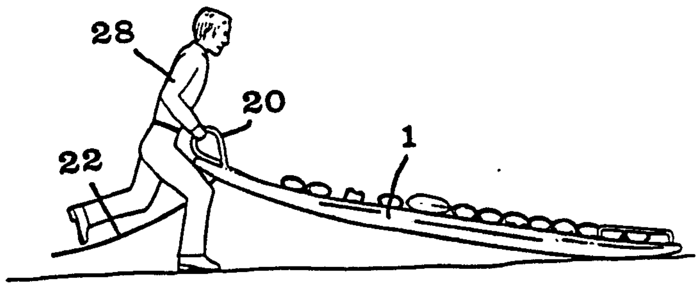


Fig 3

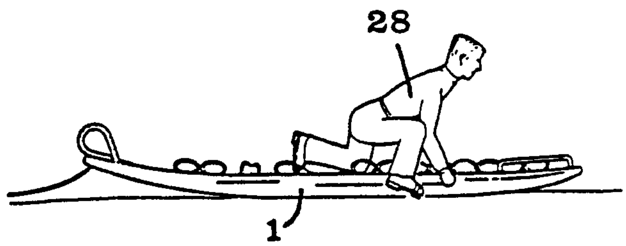


Fig 4

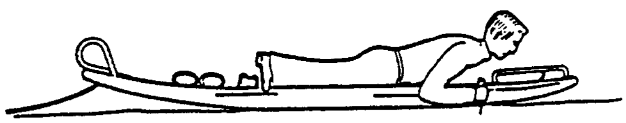

Fig 5

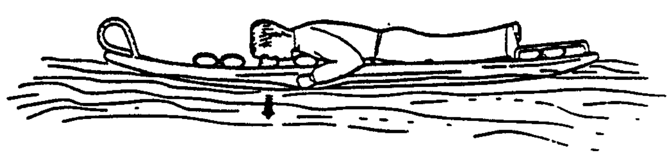

Fig 12

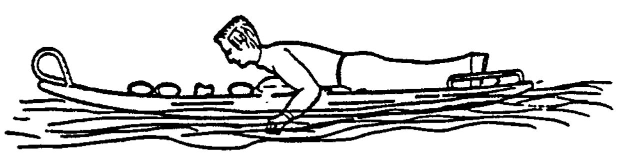

Fig 13

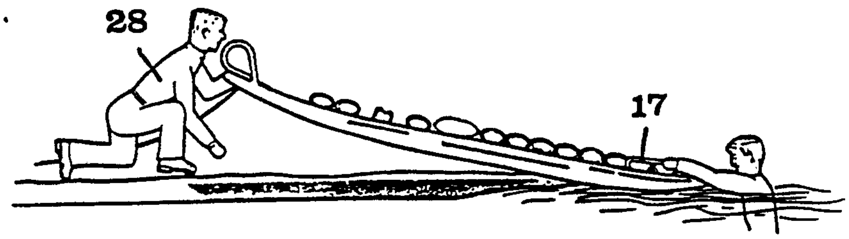


Fig 6

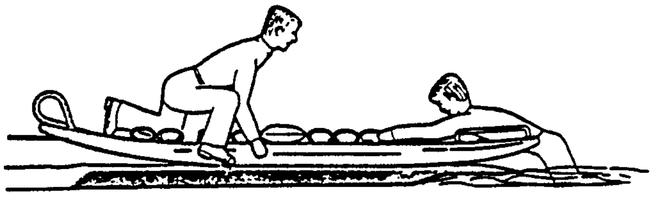

Fig 7

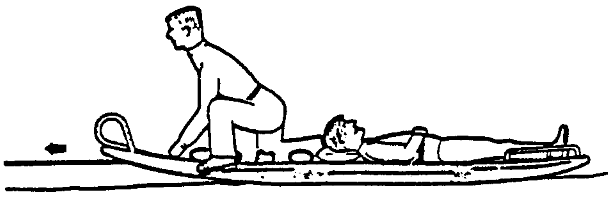

Fig 8

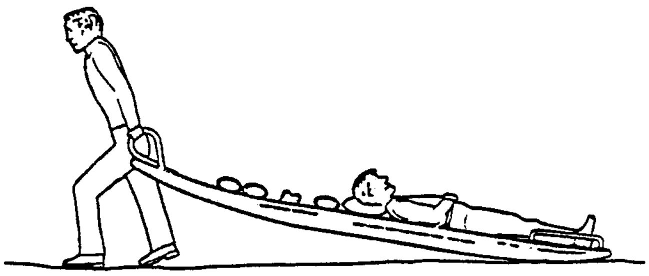

Fig 9

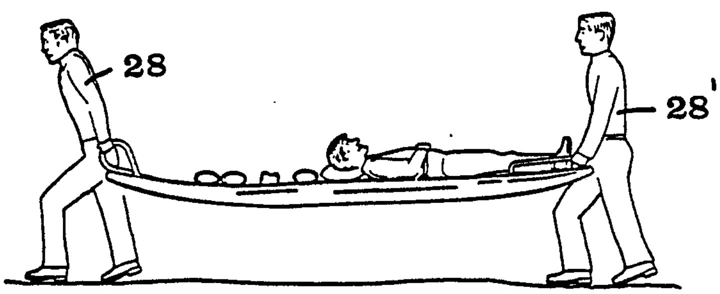


Fig 10

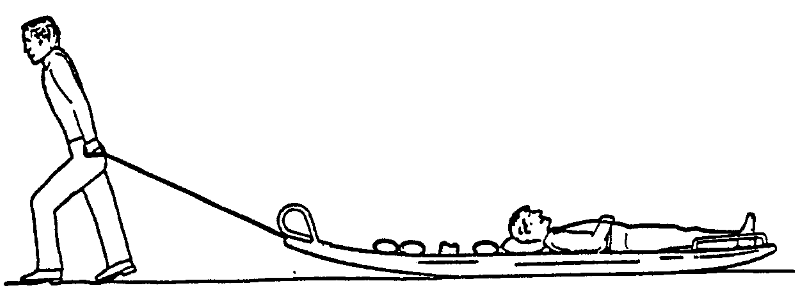

Fig 11

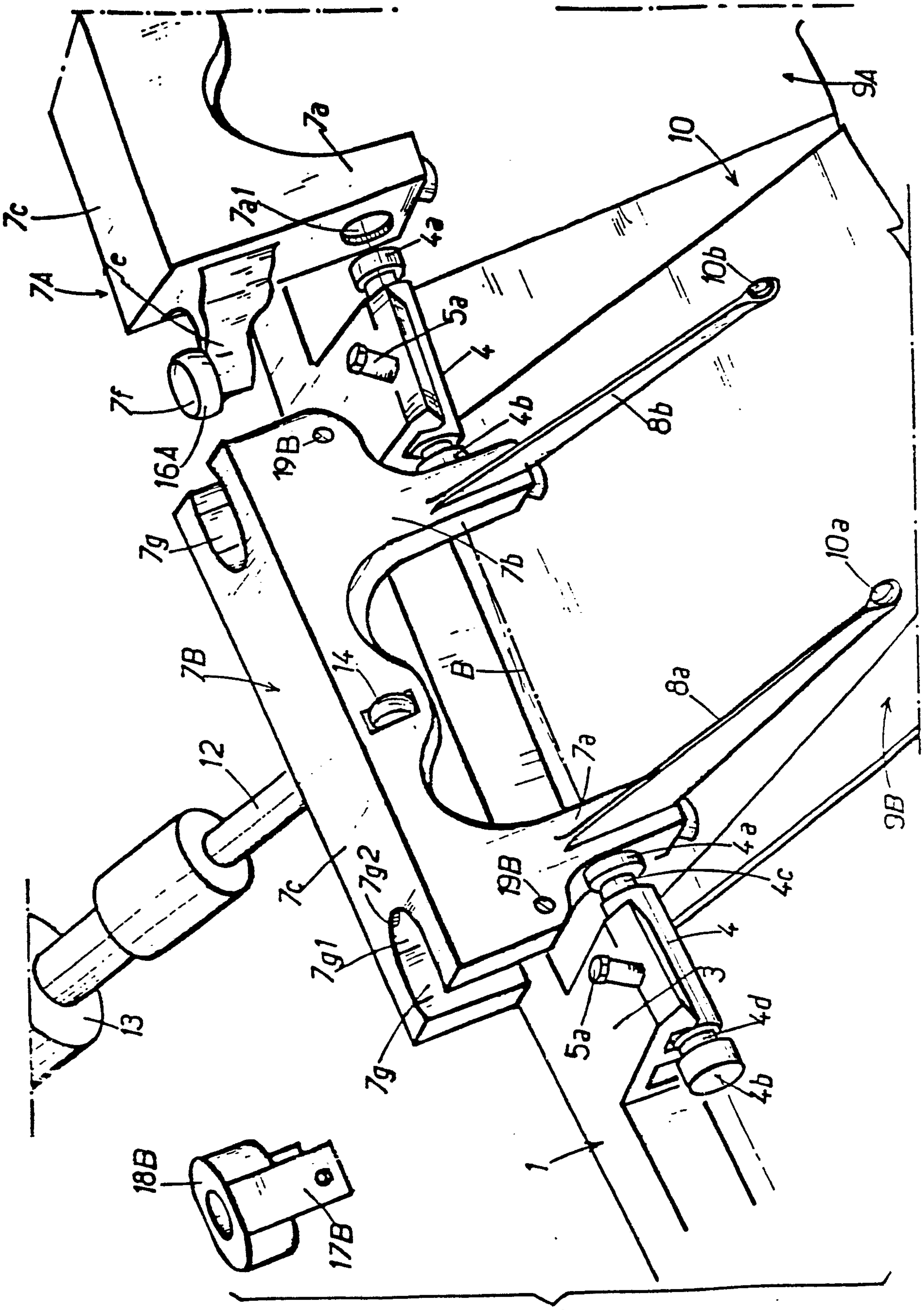
7c
7A
e
7a
7a1
4a
5a
4
10
9A
10b
8b
4b
19B
7f
16A
7g
7b
7B
14
B
10a
8a
7a
12
7c
7g2
7g1
7g
19B
4a
4c
4
3
5a
4d
4b
13
9B
1
18B
17B

FIG.1

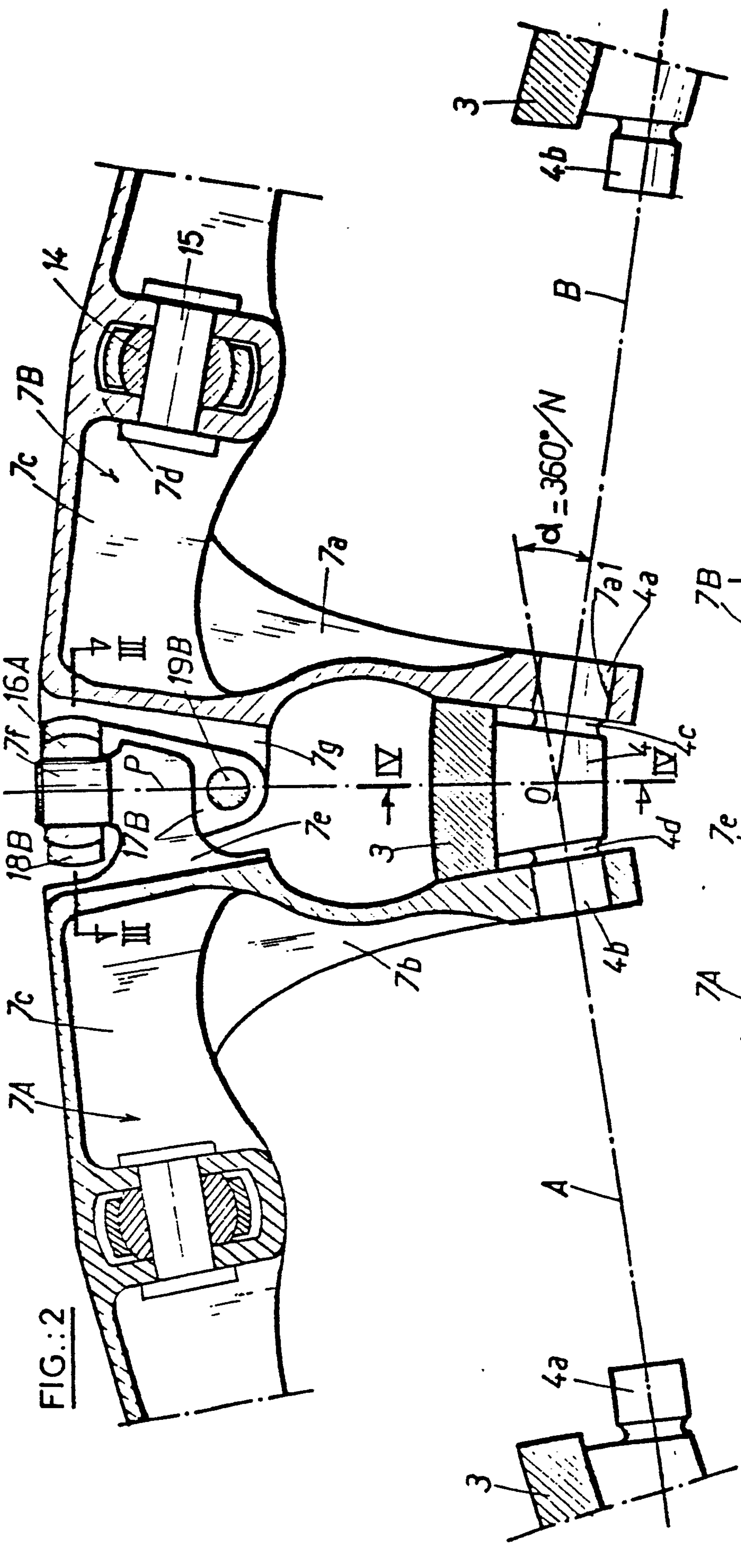

3
4b
15
14
B
7B
7c
7d
d = 360°/N
7a
7a1
4a
III
19B
16A
7f
P
7g
IV
4c
O
7B
7e
4d
3
18B
17B
III
7b
4b
7c
7A
A
4a
3


FIG.:2

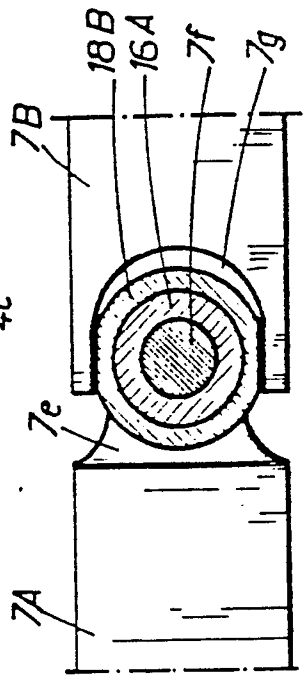

7B
18B
16A
7f
7g
7e
7A


FIG.:3

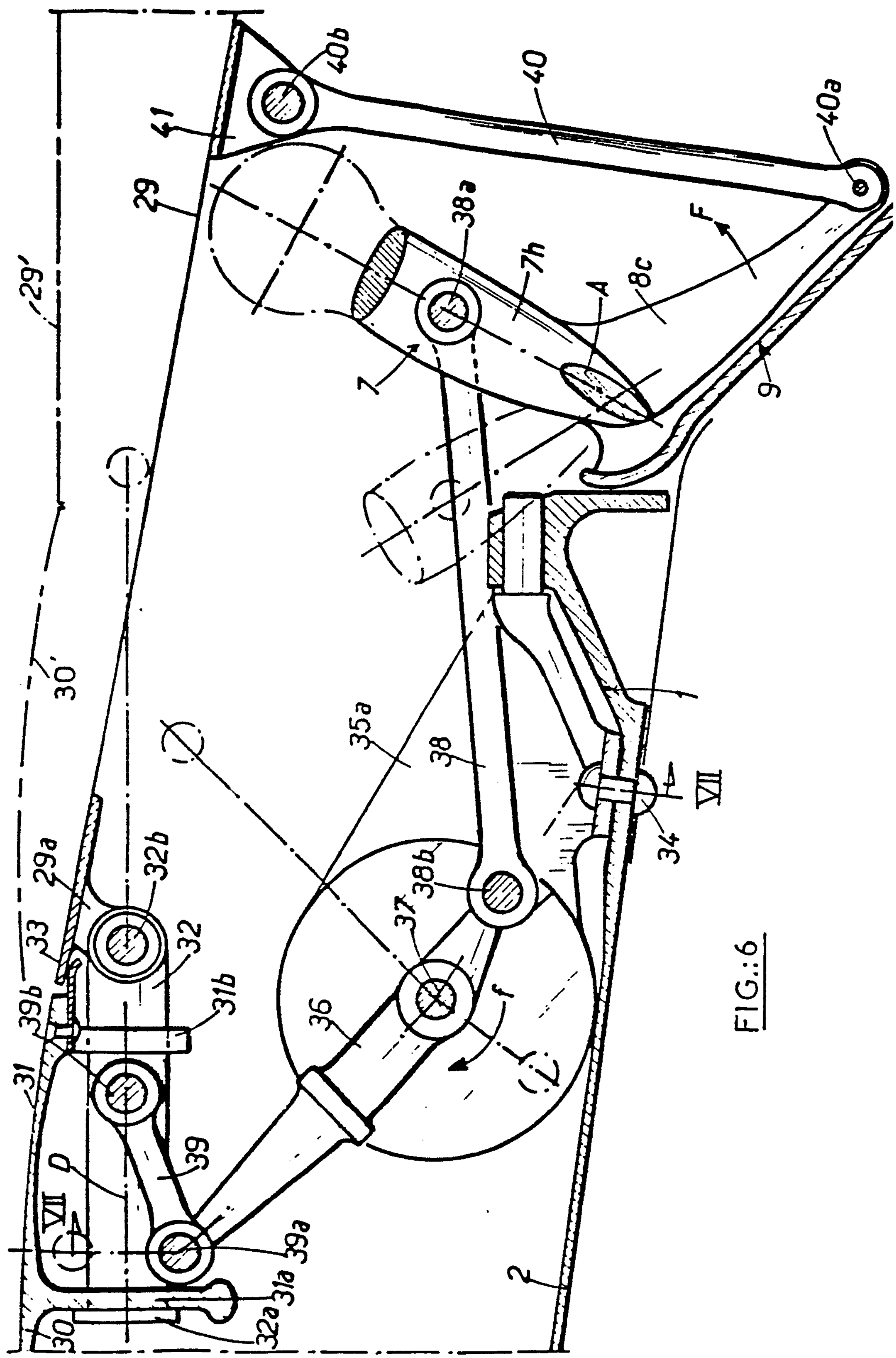
40b
40
40a
41
29
29'
38a
7h
F
8c
A
7
9
30'
35a
38
VII
34
29a
32b
33
32
38b
37
39b
31b
36
f
31
39
D
VII
39a
31a
32a
30
2

FIG.:6

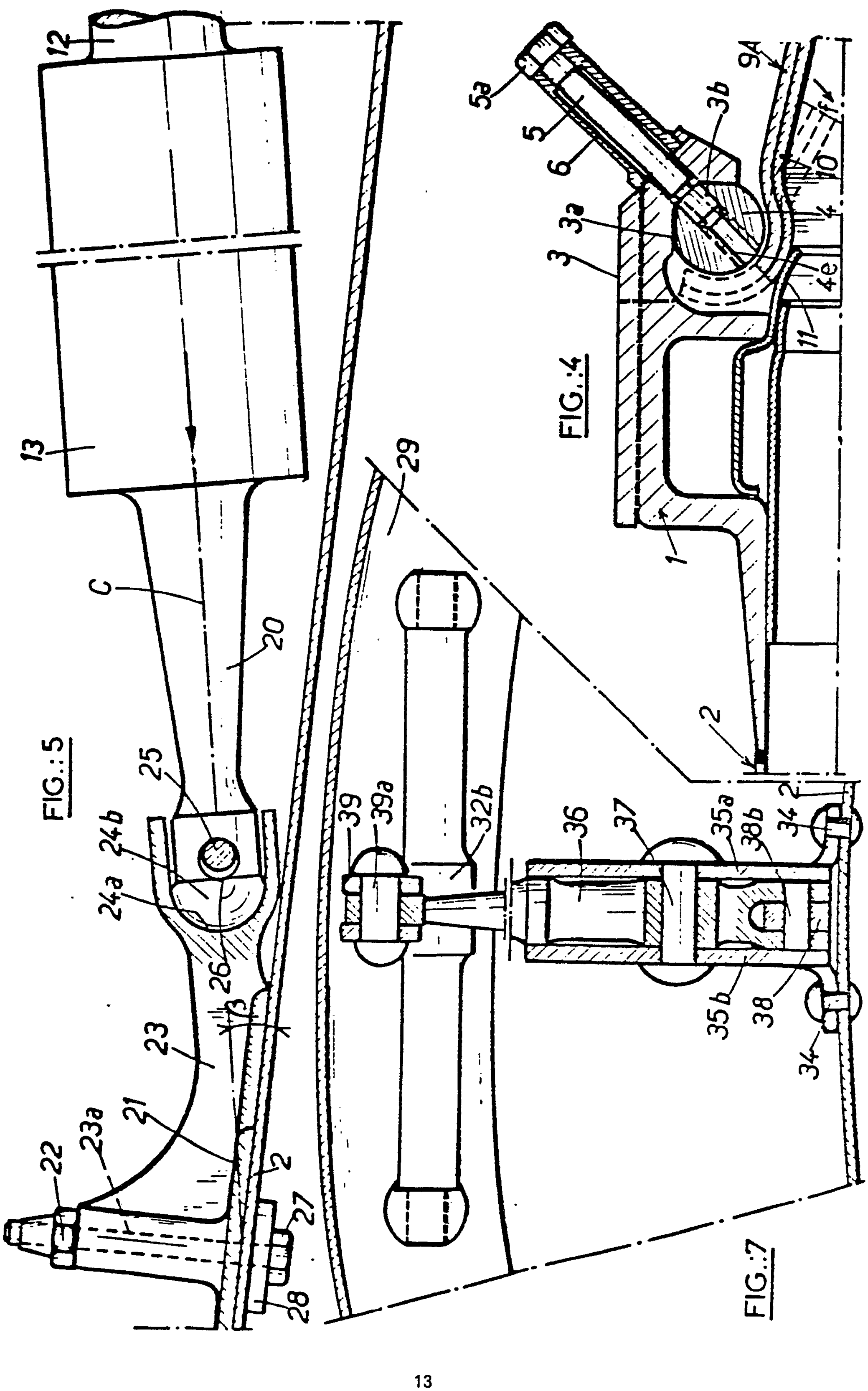
FIG.:5
FIG.:4
FIG.:7
12
13
C
20
25
24b
24a
26
23
23a
22
21
2
27
28
β
29
5a
5
6
3b
3a
3
9A
f
10
4
4e
11
1
2
36
37
35a
38b
34
35b
38
34
39
39a
32b